# EUROPEAN PATENT APPLICATION

(11) **EP 1 712 545 A1**
(43) Date of publication of application: **18.10.2006**
(21) Application number: 06007913.4
(22) Date of filing: 13.04.2006
(51) Int. Cl.: C07C 243/38

(54) **Hydroxynaphthalenedicarboxylic acid hydrazide dimer and derivatives thereof as well as process for preparing them**

(30) Priority: 14.04.2005 JP 2005116810
(71) Applicant: Kabushiki Kaisha Ueno Seiyaku Oyo Kenkyujo, Osaka-shi, Osaka-fu (JP)
(72) Inventor: Wakamori, Hiroyuki, Tanba-shi Hyogo-ken (JP)
(74) Representative: Albrecht, Thomas

(57) **Abstract**

The present invention provides a hydroxynaphthalenedicarboxylic acid hydrazide dimer or a derivative thereof represented by formula (1): 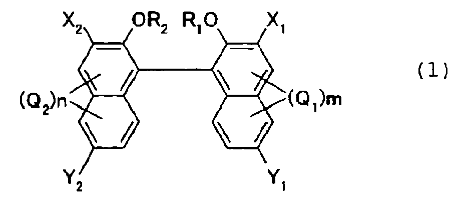 wherein X₁ and X₂ are independently selected from the group consisting of carboxyl group, a group represented by formula (2) and a group represented by formula (3):

-CO-NH-Z (2)

-CO-NHNH₂ (3)

Y₁ and Y₂ are independently selected from the group consisting of carboxyl group, carbamoyl group, a group represented by formula (2), a group represented by formula (3) and a group represented by formula (4):

-CO-O-A (4)

provided that at least one of X₁, X₂, Y₁ and Y₂ is a group represented by formula (3).

## Description

### Technical Field

The present invention relates to a novel hydroxynaphthalenedicarboxylic acid hydrazide dimer and derivatives thereof as well as a process for preparing the same.

### Background art

Hydroxynaphthalenecarboxylic acids such as 2-hydroxy-3-naphthoic acid, 2-hydroxy-6-naphthoic acid and 2-hydroxynaphthalene-3,6-dicarboxylic acid are widely used for synthesis of various products such as organic coloring matters and synthetic resins.

Some of 2-hydroxy-3-naphthoic acid compounds and their hydrazide derivatives have been used as synthetic raw materials of coupler components of azo compounds which are used as charging materials of electrophotographic photo conductors (see Japanese patent Application Laid Open Number Hei 6-95403), as rubber additives for tires or synthetic intermediates thereof (see Japanese patent Application Laid Open Numbers Hei 4-136048 and Hei 11-292834) and as curing agents or hardening accelerators for epoxy resins(see Japanese patent Application Laid Open Number Hei 9-67466). These cited references are herein incorporated by reference.

To the date, neither 2-hydroxynaphthalene-3,6-dicarboxylic acid hydrazide dimer nor its derivative is known. 2-Hydroxynaphthalene-3,6-dicarboxylic acid hydrazide dimer or its derivative may be useful as a synthetic raw material of a novel rubber additive and as a novel curing agents or hardening accelerators for epoxy resins.

### Disclosure of the invention

An object of the present invention is to provide a novel hydroxynaphthalenedicarboxylic acid hydrazide dimer and a derivative thereof as well as a process for preparing the same.

The present invention provides a hydroxynaphthalenedicarboxylic acid hydrazide dimer or a derivative thereof represented by formula (1): 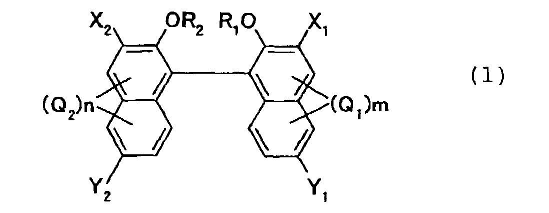 wherein X₁ and X₂ are independently selected from the group consisting of carboxyl group, a group represented by formula (2) and a group represented by formula (3):

-CO-NH-Z (2)

-CO-NHNH₂ (3)

- wherein Z is a group selected from the group consisting of an optionally branched, optionally substituted, saturated or unsaturated aliphatic group having 1-20 carbon atoms, an optionally substituted aromatic group and an optionally substituted heterocyclic group having conjugated double bonds;

Y₁ and Y-₂ are independently selected from the group consisting of carboxyl group, carbamoyl group, a group represented by formula (2), a group represented by formula (3) and a group represented by formula (4):

-CO-O-A (4)

wherein A is alkyl group having 1-6 carbon atoms;
provided that at least one of X₁, X₂, Y₁ and Y₂ in formula (1) is a group represented by formula (3);
R₁ and R₂ are independently selected from the group consisting of hydrogen atom, an optionally branched alkyl group having 1-20 carbon atoms which may be optionally substituted with hydroxyl group and/or halogen atom, and aralkyl group having 7-11 carbon atoms;
Q₁ and Q₂ are independently selected from the group consisting of alkyl group having 1-6 carbon atoms, alkoxy group having 1-6 carbon atoms, halogen atom, nitro group and hydroxyl group;
m and n represent integers of 0-4;
provided that when m or n is from 2 to 4, each of the Q₁ₛ and the Q₂ₛ may be the same or different.

In the specification and claims attached herewith, the term "aromatic group" represents a 6-membered monocyclic aromatic group or a condensed ring group consisting of up to 4 of the condensed aromatic rings.
"Heterocyclic group having conjugated double bonds" represents a 5- or 6-membered monocyclic group or a condensed ring group having at least one heteroatom selected from N, S and O and conjugated double bonds. When it constitutes a condensed ring group, said group may have up to 6 rings.

In a hydroxynaphthalenedicarboxylic acid hydrazide dimer or a derivative thereof of the present invention represented by formula (1) wherein at least one of X₁, X₂, Y₁ and Y₂ is a group represented by formula (2), examples of the X₁, X₂, Y₁ and Y₂ include alkylaminocarbonyl group, naphthylaminocarbonyl group, phenylaminocarbonyl group and the like.

In the present compounds wherein Z in formula (2) is an optionally branched, optionally substituted, saturated or unsaturated aliphatic group having 1-20 carbon atoms, examples of the Z include methyl group, ethyl group, n-propyl group, isopropyl group, n-butyl group, n-hexyl group, n-octyl group, n-nonyl group, n-decyl group, n-dodecyl group, n-hexadecyl group, n-octadecyl group, 2-chloroethyl group, vinyl group, allyl group and the like.

In the present compounds wherein Z in formula (2) is an optionally substituted aromatic group, examples of the Z include benzene ring, naphthalene ring, anthraquinone ring and the like. In the present compounds wherein Z in formula (2) is an optionally substituted heterocyclic group having conjugated double bonds, examples of the Z include thiophene, furan, pyrrole, imidazole, pyrazole, isothiazole, isoxazole, pyridine, pyrazine, pyrimidine, pyridazine, triazole, tetrazole, indole, 1H-indazole, purine, 4H-quinolizine, isoquinoline, quinoline, phthalazine, naphthyridine, quinoxaline, quinazoline, cinnoline, pteridine, benzofuran and the like.

Examples of substituents on Z include halogen atom, halogenated C₁₋₆ alkyl, nitro group, C₁₋₆ alkyl group, C₁₋₆ alkoxy group (such as methoxy group), cyano group, phenoxy group, phenylamino group, benzoylamino group, penylcarbamoyl group, alkylaminosulfonyl group and C₂₋₆ alkenyl group optionally having aryl group and the like. When the substituent on Z contains aromatic ring group, said ring may have one or more further substituents such as halogen atom, C₁₋₆ alkyl group, C₁₋₆ alkoxy group, phenyl group, cyano group and the like.

In the compound of the present invention represented by formula (1) wherein at least one of Y₁ and Y₂ is a group represented by formula (4), examples of -the Y₁ and Y₂ include methoxycarbonyl group, ethoxycarbonyl group, n-butoxycarbonyl group and the like.

In a hydroxynaphthalenedicarboxylic acid hydrazide dimer or a derivative thereof represented by formula (1) of the present invention, R₁ and R₂ are independently selected from the group consisting of hydrogen atom, an optionally branched alkyl group having 1-20 carbon atoms which may be optionally substituted with hydroxyl group and/or halogen atom, and aralkyl group having 7-11 carbon atoms.

In the compound of the present invention wherein at least one of R₁ and R₂ is an optionally branched alkyl group having 1-20 carbon atoms which may be optionally substituted with hydroxyl group and/or halogen atom, examples of the R₁ and R₂ include ethyl group, n-propyl group, isopropyl group, n-butyl group, isobutyl group, t-butyl group, n-pentyl group, n-hexyl group, n-octyl group, n-nonyl group, n-dodecyl group, n-hexadecyl group, n-octadecyl group, 2-hydroxyethyl group, 2-chloroethyl group, 4-hydroxybutyl group and the like. In the compound of the present invention wherein at least one of R₁ and R₂ is an aralkyl group having 7-11 carbon atoms, examples of the R₁ and R₂ include benzyl group, phenethyl group, phenylpropyl group, 1-naphthylmethyl group, 2-naphthylmethyl group and the like.

In a hydroxynaphthalenedicarboxylic acid hydrazide dimer or a derivate thereof of the present invention represented by formula (1), Q₁ and Q₂ represent substituents on the naphthalene rings. Examples of the Q₁ and Q₂ include C₁₋₆ alkyl group, C₁₋₆ alkoxy group, halogen atom, nitro group and hydroxyl group- In formula (1) of the present invention, m and n represent integers of 0-4, which are the number of substituents on the corresponding naphthalene rings. When at least one of m and n represent 2-4, i.e., when at least one of the naphthalene rings is substituted with two or more substituents, the Q₁ₛ and the Q₂ₛ may be the same or different.

In the synthesis of a hydroxynaphthalenedicarboxylic acid hydrazide dimer or a derivative thereof represented by formula (1) of the present invention, a derivative of hydroxynaphthalenedicarboxylic acid dimer represented by formula (5), the starting material, may be prepared by any known method: 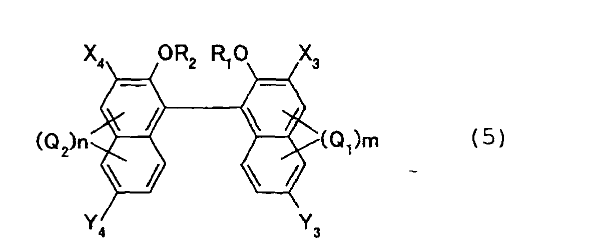 wherein X₃, X₄, Y₃ and Y₄ are independently selected from the group consisting of a group represented by formula (2), a group represented by formula (3), a group represented by formula (4), carbamoyl group and carboxyl group;
provided that at least one of X₃, X₄, Y₃ and Y₄ is a group represented by formula (4) or carbamoyl group;
R₁, R₂, Q₁, Q₂, m and n are the same as defined above with regard to formula (1).

Some methods for preparing a compound of formula (5) are described in, for example, Japanese patent Application Laid Open Numbers 2002-316961 and 2001-316966. These cited references are herein incorporated by reference. A schematic illustration of these methods is shown in scheme 1 below.

Specifically, one example of methods for preparing a compound of formula (5) comprises coupling a compound of formula (6) with a compound of formula (7) in solvent such as N,N-dimethylformamide in the presence of cuprous chloride (CuCl), separating a compound represented by formula (8) by conventional methods such as chromatograph and then, alkylating or aralkylating the resulting compound of formula (8). The separation step is required because dimers of compounds of formula (6) and those of compounds of formula (7), side-products, may be produced in the coupling reaction.

With regard to compounds of formula (6) and formula (7), each of X₃, Y₃, X₄ and Y₄ represents a group other than carboxyl group. If one or more of X₃, Y₃, X₄ and Y₄ represents carboxyl group, the coupling reaction is inhibited due to the binding of cuprous salt to carboxyl group.

An example of methods for preparing a compound of formula (5) wherein one to three of groups selected from X₃, Y₃, X₄ and Y₄ is carboxyl group comprises preparing a compound of formula (8) having one to three groups represented by formula (4) and hydrolyzing the groups represented by formula (4) according to a conventional method.

Preferable compounds represented by formula (5) are the compounds wherein X₃, Y₃ , X₄ and Y₄, R₁ and R₂ , Q₁ and Q₂ as well as m and n are respectively the same. 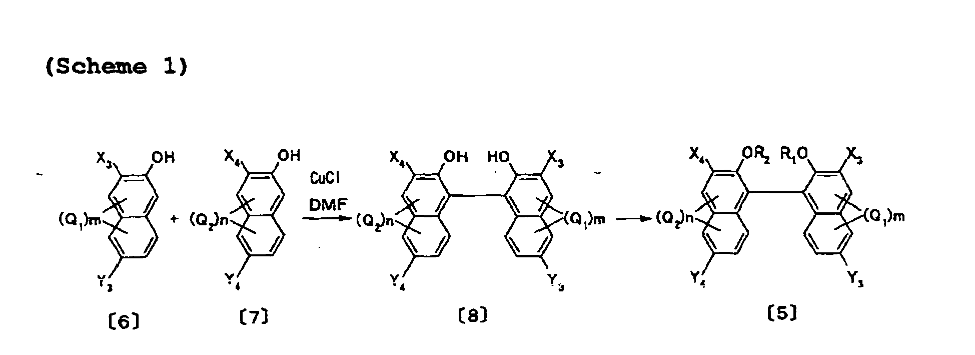 wherein X₃, Y₃, X₄ and Y₄ in formulae (6)-(8) are selected from the group consisting of a group represented by formula (2), a group represented by formula (3), a group represented by formula (4) and carbamoyl group, and at least one of X₃, Y₃, X₄ and Y₄ is a group represented by formula (4) or carbamoyl group;
Q₁, Q₂, m and n are the same as defined above with regard to formula (5);
provided that 1-positions of the naphthalene rings of both compounds represented by formula (6) and formula (7) are hydrogen atoms.

A hydroxynaphthalene dicarboxylic acid derivative represented by formula (6) or formula (7), the starting material in the scheme 1, may be obtained by any known method. A method for preparing a compound of formula (6) is described herein below in detail, and a compound of formula (7) may be prepared by the same method as that for preparing a compound of formula (6). 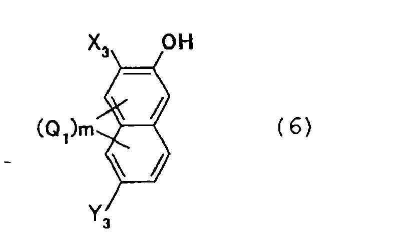 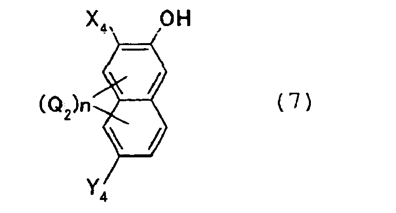 wherein X₃, Y₃, X₄ and Y₄ in formulae (6) and (7) are selected from the group consisting of a group represented by formula (2), a group represented by formula (3), a group represented by formula (4) and carbamoyl group, and at least one of X₃, Y₃, X₄ and Y₄ is a group represented by formula (4) or carbamoyl group;
Q₁, Q₂, m and n are the same as defined above with regard to formula (1);
provided that 1-positions of the naphthalene rings of both compounds represented by formula (6) and formula (7) are hydrogen atoms.

For example, a compound of formula (6) wherein both of X₃ and Y₃ are formula (4):

-CO-O-A (4)

wherein A represents alkyl group having 1-6 carbon atoms, or carbamoyl group may be prepared by the method illustrated in the following scheme 2:

Specifically, 2-hydroxynaphthalene-3,6-dicarboxylic acid or a derivative thereof represented by formula (9) is reacted with thionyl chloride in solvent such as tetrahydrofuran to give an acid chloride represented by formula (10), which is reacted with a C₁₋₆ alcohol represented by A-OH to give an ester derivative of the hydroxynaphthalenedicarboxylic acid represented by formula (11). If the acid chloride represented by formula (10) is reacted with ammonia, a derivative of the hydroxynaphthalenedicarboxylic acid having carbamoyl group represented by formula (12) may be obtained. 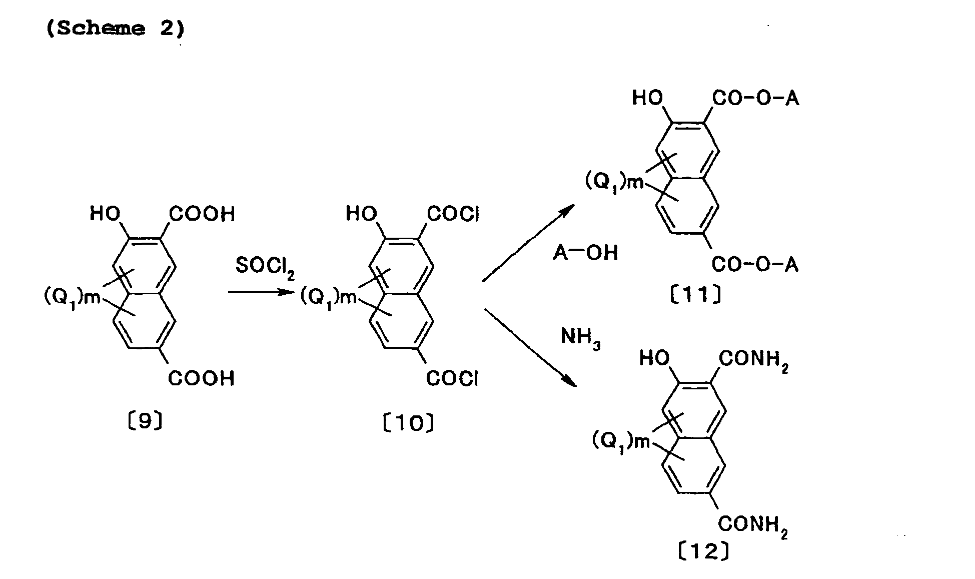 wherein Q₁ and m in formulae (9)-(12) are the same as defined with regard to formula (1). A-OH represents C₁₋₆ alcohol.

A compound represented by formula (11) may also be prepared by reacting a compound represented by formula (9) with A-OH, i.e., C₁₋₆ alcohol in the presence of acid such as sulfuric acid, para-toluenesulfonic acid and the like.

In case where a compound represented by formula (9) is 2-hydroxynaphthalene-3,6-dicarboxylic acid, the compound may be prepared according to the method disclosed in WO98/17621. The cited reference is herein incorporated by reference. 2-Hydroxynaphthalene-3,6-dicarboxylic acid derivatives represented by formula (9) may be prepared according to a conventional method which comprises introducing one or more substituent on the naphthalene ring.

In the present invention, a derivative of a hydroxynaphthalenedicarboxylic acid represented by formula (6) wherein one of X₃ and Y₃ is a group represented by formula (2) or carbamoyl group and the other is a group represented by formula (4) may be prepared by obtaining a compound represented by formula (15) or formula (17) according to scheme 3-1 or 3-2 and then reacting the compound represented by formula (15) or formula (17) with thionyl chloride to give the compound having chlorocarbonyl group according to scheme 3-3 or 3-4. Then the compound having chlorocarbonyl group is reacted with an appropriate amine or ammonia to give a compound represented by any one of formulas (19), (20), (22) and (23).

The reason why the selective esterification and hydrolysis of 3-position on the naphthalene ring is achieved as shown in schemes 3-1 and 3-2 is that the influence of the hydroxyl group on 2-position of the naphthalene ring causes the higher reactivity of 3-position than that of 6-position.

In the present invention, a hydroxynaphthalene dicarboxylic acid derivative represented by formula (6) wherein one of X₃ and Y₃ is a group represented by formula (2) and the other is carbamoyl group may be prepared by hydrolyzing a group represented by formula (4) of a compound of formula (19) or formula (22) to give carboxyl group and thereafter, amidating thus obtained carboxyl group with an amine represented by Z-NH₂ according to a conventional method. Alternatively, a compound of formula (20) or (23) having a group of formula (4) is hydrolyzed to give a compound having a carboxyl group and then the resultant compound is reacted with ammonia to give a compound having carbamoyl group.

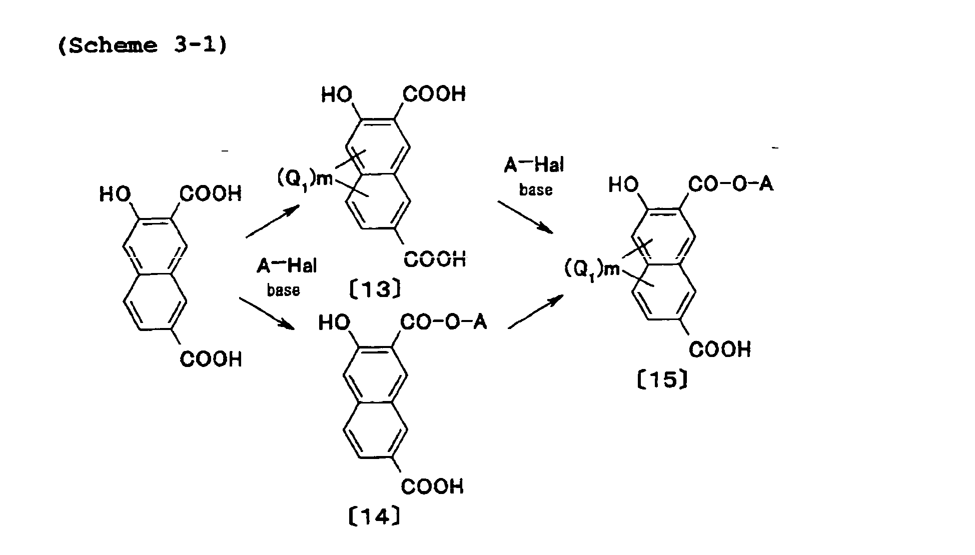

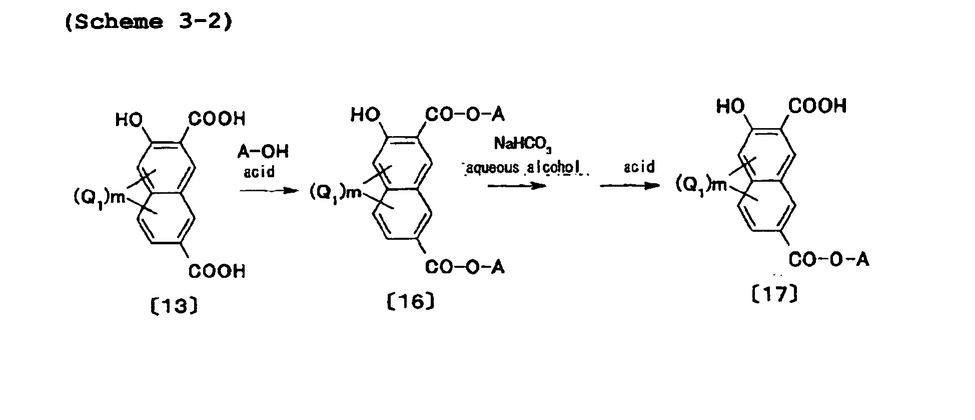

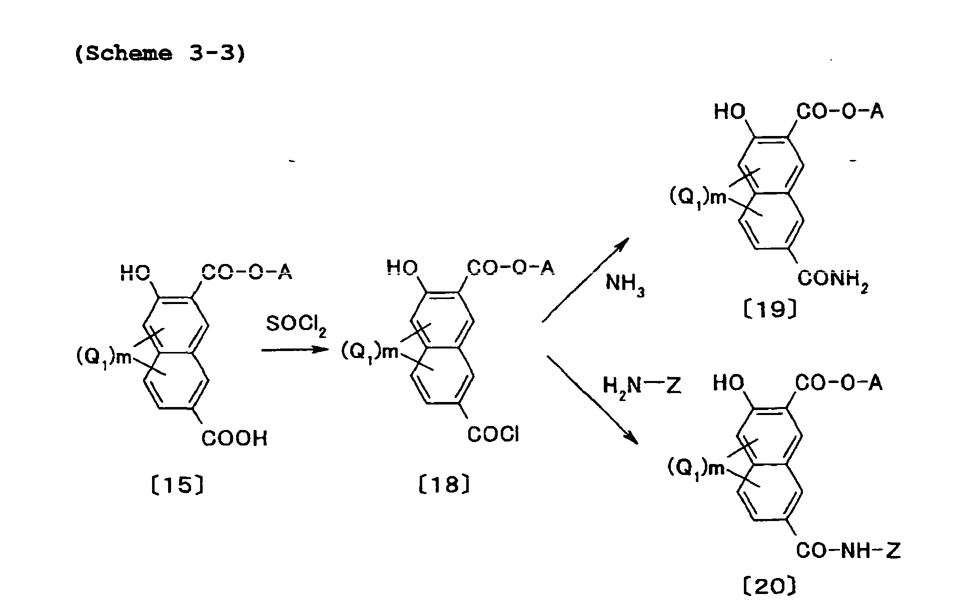

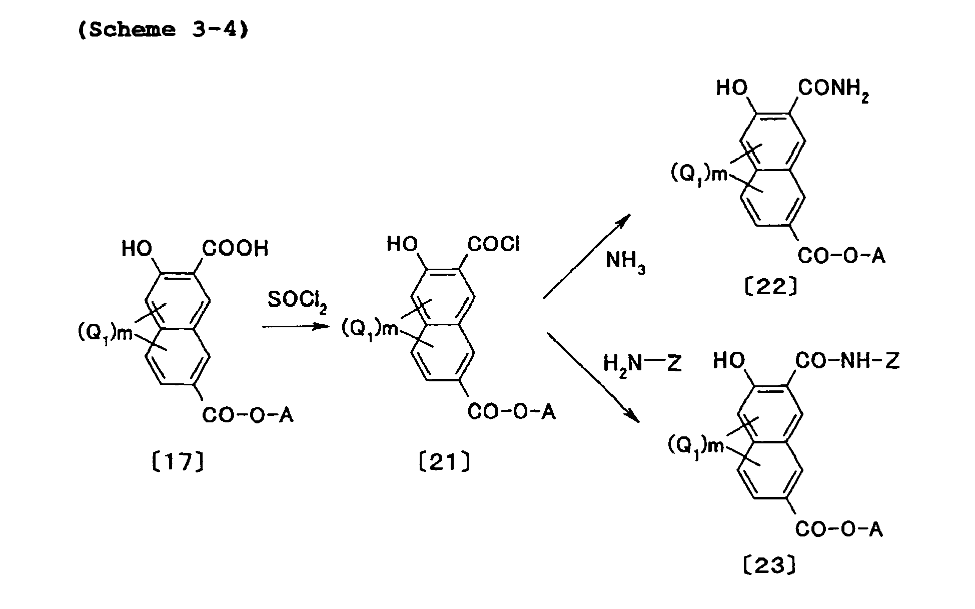

In formulae (13)-(23), Q₁ and m are the same as defined with regard to formula (1). A-OH represents C₁₋₆ alcohol and A-Hal represents C₁₋₆ halogenated alkyl. H₂N-Z represents an amine which is selected from the group consisting of an optionally branched, optionally substituted saturated or unsaturated aliphatic amine having 1-20 carbon atoms, an optionally substituted aromatic amine and an optionally substituted heterocyclic amine having conjugated double bonds.

The hydroxynaphthalenedicarboxylic acid dimer derivative represented by formula (5) obtained as above is reacted with at least one hydrazine compound which is selected from the group consisting of hydrazine monohydrate, hydrazine sulfate, dihydrazine sulfate, hydrazine monohydrochloride, hydrazine dihydrochloride and hydrazine hydrobromide to give a hydroxynaphthalenedicarboxylic acid hydrazide dimer or a derivative thereof.

Among the above hydrazine compounds, hydrazine monohydrate is preferably used because its side-product is water, which may be easily removed.

The amount of a hydrazine compound used for the reaction may preferably be 1.2-5.0 moles, and more preferably 2.0-2.5 moles per one mole of the total amount of the group represented by formula (4) and carbamoyl group which are present in a hydroxynaphthalenedicarboxylic acid dimer derivative represented by formula (5).

The temperature of the reaction of the hydroxynaphthalenedicarboxylic acid dimer derivative represented by formula (5) with a hydrazine compound may preferably be 20-110°C and more preferably 80-100°C for high reaction rate and less side product.

Solvents used for the hydrazidation reaction are not limited as long as they are inactive to the reaction. Examples of such solvents include alcohols such as methanol, ethanol, n-propanol, n-butanol and 2-ethylhexyl alcohol.

The hydrazidation reaction may be carried out until no less than 80 mole %, preferably no less than 90 mole % and more preferably no less than 95 mole % of the hydroxynaphthalenedicarboxylic acid dimer derivative represented by formula (5), the starting material, is converted to the hydrazide derivative. The conversion rate can be confirmed with HPLC and the like.

The reaction time may vary depending on the reaction temperature and the solvent used, and in general, it may be 5-100 hours.

The hydrazidation reaction may be conducted by any manner known to the art and may be by means of batchwise reaction or continuous reaction.

In the present invention, if the starting material of the hydrazidation reaction is a compound represented by formula (5) wherein R₁ and/or R₂ is hydrogen atom (i.e., 2- and/or 2'-posotion of the naphthalene rings is hydroxy group), the reactivity of a group represented by formula (4) or that of carbamoyl group on 6- and/or 6'-position of the naphthalene rings may be decreased.

In such cases, the compound represented by - formula (1) wherein R₁ and/or R₂ is hydrogen atom and 6- and/or 6'-position of the naphthalene rings is represented by formula (3) may be prepared by the method which comprises conducting the hydrazidation reaction using a compound represented by formula (5) wherein R₁ and/or R₂ is alkyl or aralkyl group as a starting material to give a compound having hydrazide group on 6- and/or 6'-position. And then the alkyl or aralkyl group of said compound is removed according to a conventional method.

After the hydrazidation reaction is completed, the hydroxynaphthalenedicarboxylic acid hydrazide dimer or derivative thereof represented by formula (1) is precipitated, for example, by cooling, concentrating or adding poor solvent such as water. Thereafter, the precipitate is separated from the reaction mixture by any known means such as centrifugation and filter press and then dried.

The resulting hydroxynaphthalenedicarboxylic acid hydrazide dimer or derivative thereof represented by formula (1) may be purified by recrystallization or washing it with organic solvent and/or water as desired. When one or more substituent selected from the group consisting of C₁₋₆ alkyl group, C₁₋₆ alkoxy group, halogen atom, nitro group and hydroxyl group can be introduced on the naphthalene rings. The substituents may be introduced according to a conventional method.

The hydroxynaphthalenedicarboxylic acid hydrazide dimer or derivative thereof obtained by the process of the present invention may suitably be used as a rubber additive for tires and a curing agent/hardening accelerator for epoxy resins or as a synthetic raw material for these products.

### Brief Description of the Drawings

Figure 1 is an infrared absorption spectrum of the compound of formula (I) obtained in Example 1.
Figure 2 is an infrared absorption spectrum of the compound of formula (II) obtained in Example 2.

The present invention is further described in reference to the following examples. The examples are intended to illustrate the invention and not to be construed to limit the scope of the invention.

### Example 1

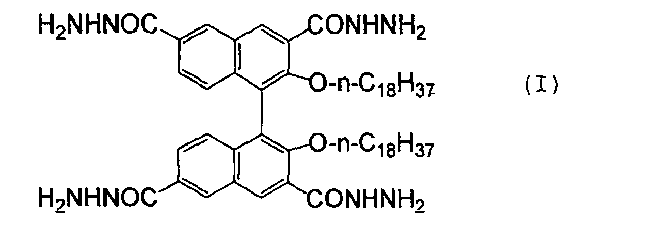

40.9 g (40 mmol) of 1,1'-Bis(2-stearyloxy-3,6-din-butoxycarbonylnaphthalene) obtained by a known method was suspended in 100 g of n-butanol and to this mixture, 17.3 g (275 mmol) of hydrazine monohydrate was added dropwise at room temperature.

The reaction mixture was heated from room temperature to 100°C over one hour and kept at this temperature with stirring. At this temperature, the hydrazidation reaction was carried out for 20 hours and the resulting reaction mixture was analyzed by HPLC, which confirmed that the conversion rate of 1,1'-bis(2-stearyloxy-3,6-di-n-butoxycarbonylnaphthalene) was no less than 95 mol %. The reaction mixture was cooled to room temperature and the hydrazidation reaction was completed.

The precipitate was separated from the reaction mixture by means of suction filtration to give the crude crystal of 1,1'-bis{2-stearyloxy-3,6-di-(hydrazinocarbonyl) naphthalene}. The resulting crude crystal was washed by suspending it in 80 g of cold methanol, filtrated and dried to give 22.1 g (21.6 mmol) of the white crystal of 1,1'-bis{2-stearyloxy-3,6-di-(hydrazinocarbonyl)naphthalene}.

Mass spectrum: m/z (-) 1022, m/z (+) 1046 (+Na+) (MW 1023.4)

Melting point: 174°C

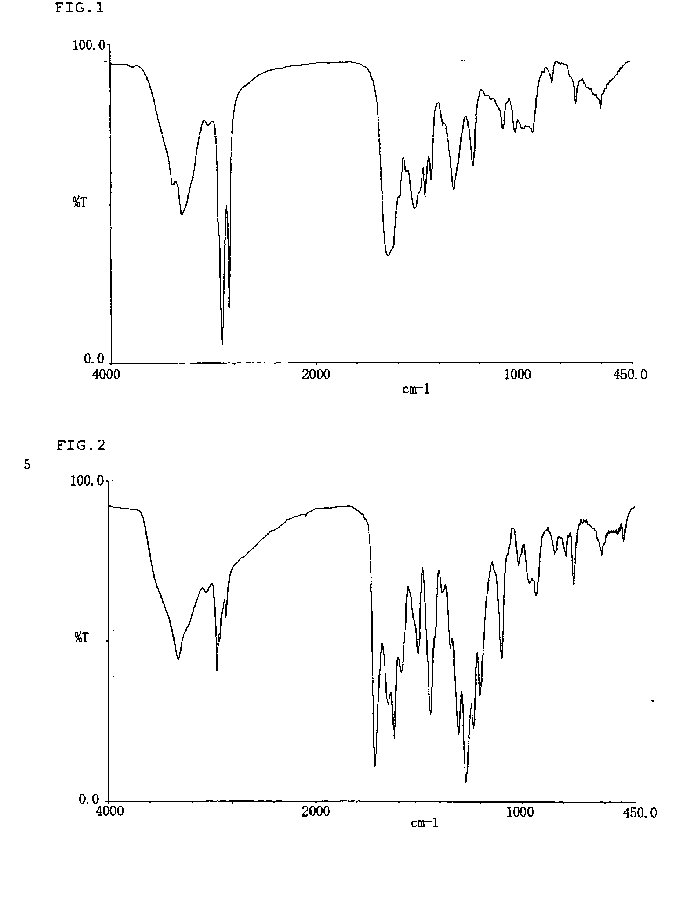

The infrared absorption spectrum (KBr method) of the resulting 1,1'-bis{2-stearyloxy-3,6-di-(hydrazinocarbonyl)naphthalene} is shown in Figure 1.

### Example 2

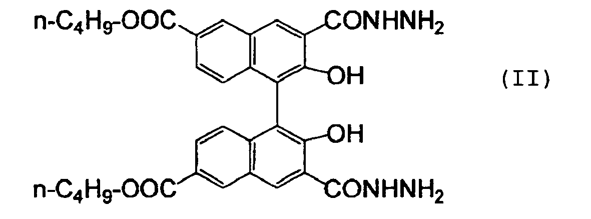

According to the same manner as described in Example 1 with the exception that 41.2 g (60 mmol) of 1,1'-bis(2-hydroxy-3,6-di-n-butoxycarbonylnaphthalene) was used in stead of 40.9 g (40 mmol) of 1,1'-bis(2-stearyloxy-3,6-di-n-butoxycarbonylnaphthalene) and the reaction was carried out for two days in stead of 20 hours, 32.9 g (54.6 mmol) of the white crystal of 1,1'-bis(2-hydroxy-6-n-butoxycarbonyl-3-hydrazinocarbonylnaphthalene) was obtained.

Mass spectrum: m/z (-) 601, m/z (+) 603 (MW 602.6)

Decomposition point: 270°C

The infrared absorption spectrum (KBr method) of the resulting 1,1'-bis(2-hydroxy-6-n-butoxycarbonyl-3-hydrazinocarbonylnaphthalene) is shown in Figure 2.

## Claims

1. A hydroxynaphthalenedicarboxylic acid hydrazide dimer or a derivative thereof represented by formula (1): 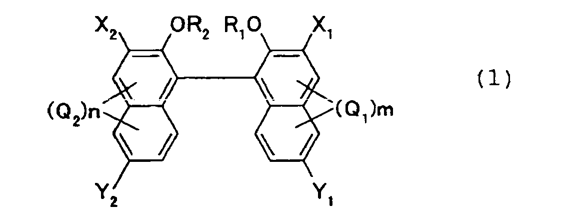 wherein X₁ and X₂ are independently selected from the group consisting of carboxyl group, a group represented by formula (2) and a group represented by formula (3):
-CO-NH-Z (2)
-CO-NHNH₂ (3)
wherein Z is a group selected from the group consisting of an optionally branched, optionally substituted, saturated or unsaturated aliphatic group having 1-20 carbon atoms, an optionally substituted aromatic group and an optionally substituted heterocyclic group having conjugated double bonds;
Y₁ and Y₂ are independently selected from the group consisting of carboxyl group, carbamoyl group, a group represented by formula (2), a group represented by formula (3) and a group represented by formula (4):
-CO-O-A (4)
wherein A is alkyl group having 1-6 carbon atoms;
provided that at least one of X₁, X₂, Y₁ and Y₂ in formula (1) is a group represented by formula (3);
R₁ and R₂ are independently selected from the group consisting of hydrogen atom, an-optionally branched alkyl group having 1-20 carbon atoms which may be optionally substituted with hydroxyl group and/or halogen atom and aralkyl group having 7-11 carbon atoms;
Q₁ and Q₂ are independently selected from the group consisting of alkyl group having 1-6 carbon atoms, alkoxy group having 1-6 carbon atoms, halogen atom, nitro group and hydroxyl group;
m and n represent integers of 0-4;
provided that when m or n is from 2 to 4, each of the Q_{1 s} and the Q_{2 s} may be the same or different.

2. A process for preparing the hydroxynaphthalenedicarboxylic acid hydrazide dimer or derivative thereof according to claim 1, which comprises: reacting a hydroxynaphthalenedicarboxylic acid dimer derivative represented by formula (5) with one or more hydrazine compound selected from the group consisting of hydrazine monohydrate, hydrazine sulfate, dihydrazine sulfate, hydrazine monohydrochloride, hydrazine dihydrochloride and hydrazine hyrdobromide at a temperature of 20-110°C: 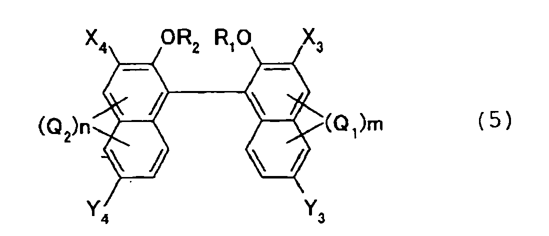 wherein X₃, X₄, Y₃ and Y₄ are independently selected from the group consisting of a group represented by formula (2), a group represented by formula (3), a group represented by formula (4), carbamoyl group and carboxyl group;
provided that at least one of X₃, X₄, Y₃ and Y₄ is a group represented by formula (4) or carbamoyl group;
R₁, R₂, Q₁, Q₂, m and n are the same as defined in claim 1.

3. The process according to claim 2, wherein said hydrazine compound is hydrazine monohydrate.
